# EUROPEAN PATENT SPECIFICATION

(11) **EP 0 406 324 B1**
(45) Date of publication and mention of the grant of the patent: **01.12.1993**
(21) Application number: 89905016.5
(22) Date of filing: 20.03.1989
(51) Int. Cl.: A61K 37/02

(54) **HEMATOPOIETIC COLONY-STIMULATING FACTORS IMPROVE THE LIPOPROTEIN CHOLESTEROL PROFILE**
HEMATOPOIETISCHE KOLONIEN STIMULIERENDE FAKTOREN ZUR VERBESSERUNG DES LIPOPROTEIN/CHOLESTERIN-PROFILS
AMELIORATION DU PROFIL DU CHOLESTEROL LIPOPROTEINIQUE PAR DES FACTEURS STIMULATEURS DE COLONIE HEMATOPOIETIQUE

(30) Priority: 21.03.1988 US 170478; 08.12.1988 US 281432
(43) Date of publication of application: 09.01.1991
(73) Proprietor: GENETICS INSTITUTE, INC., Cambridge, Massachusetts 02140 (US)
(72) Inventor: GARNICK, Marc, Brookline, MA 02146 (US)
(74) Representative: Weinhold, Peter, Dr.
(86) International application number: PCT/US89/01137
(87) International publication number: WO 89/09060

(56) References cited:
- Jama, vol. 280, no. 22, 9 Dec. 1988, S.D. Nimer et al., pp. 3297-3300
- Behring Inst. Mitt., vol. 83, Aug. 1988, R.M. Rifkin et al., pp. 125-133
- Science, vol. 239, 15 Jan. 1988, J.L. Marx, pp. 257-258
- Experimental and Molecular Pathology, vol. 36, 1982, Acad. Press, Inc., W.Dodge et al., pp. 44-56
- Science, vol. 236, June 1987, S.C. Clark et al., pp. 1229-1237

## Description

Abnormal blood cholesterol levels increase an individual's risk of coronary heart disease. Cholesterol is transported in the body largely through the interaction of the lipoproteins. For this reason a great deal of attention has focused on the search for agents that safely and effectively reduce levels of lipoprotein, other lipids, and thus cholesterol in the blood. Elevated levels of blood cholesterol increases an individual's risk of encountering coronary heart disease. While blood cholesterol levels can be somewhat reduced through diet modifications, recourse to drug therapy is often required.

Coronary heart disease (CHD) is caused by atherosclerosis which results from the formation of atherosclerotic plaques or lesions. These plaques are build-ups of cholesterol deposited in the arterial wall. The build-up occurs because cholesterol is insoluble in water and thus not readily removed by the blood. Thus, "[i]f cholesterol is to be transported safely in blood, its concentration must be kept low and its tendency to escape from the bloodstream must be controlled." M.S. Brown and J.L. Goldstein, Science, 232:34-47 (1986).

Initially, free cholesterol is bound to the surface of high-density lipoproteins (HDL) and coupled to a fatty acid in an esterification reaction. The cholesteryl esters which are formed on the surface of HDL are subsequently encapsulated within low-density lipoproteins (LDL). The cholesteryl ester-containing LDL then enters certain cells through a lipoprotein-specific receptor-mediated-endocytosis. Once inside the cell, the esters are hydrolyzed back to cholesterol and put to constructive use in the formation of steroids, membranes, or it is biochemically modified for safe and efficient removal from the body.

The LDL is the most abundant cholesterol carrying lipoprotein in the human body and carries about three-fourths of the total cholesterol of normal human plasma. Lowering plasma LDL levels will effect a reduction of serum cholesterol and so decrease the progression of atherosclerosis. Furthermore, because the HDL removes free cholesterol from the plasma and prepares it for encapsulation within the LDL an increased HDL/LDL ratio is generally accepted as representing an improved lipoprotein cholesterol profile.

The importance of maintaining a healthy overall lipoprotein cholesterol profile has recently been made evident. B. Lewis (Acta Med. Scand. (Suppl.) 701:53-57, 1985) observed a significant positive relationship between LDL cholesterol level and progession of arterial disease. Recent studies suggest that heart disease can strike even individuals having safe levels of total cholesterol if their levels of HDL are low. Wall Street Journal, (November 17, 1988, page B4). Thus, not only high levels of LDL, but also low levels of HDL can be very dangerous.

S.C. Clark and R. Kamen (Science, 236:1229-1237, 1987) review the role of the major human myeloid growth factors such as granulocyte macrophage colony stimulating factor (GM-CSF), macrophage colony stimulating factor (M-CSF) or interleukin-3(IL-3) in regulating hematopoiesis in vivo. Medical applications for colony-stimulating factors (CSFs) in the restoration of hematopoietic dysfunction, in augmentation of host defense against infection and in malignant disease are discussed.

Dodge et al., (Experimental and Molecular Pathology 36:44-56, 1982) describe the effect of hypercholesterolemia on in vitro production of granulocytes and monocytes. It was shown that LDL from hypercholesterolemic chicks markedly inhibited the in vitro replication of macrophage progenitor cells.

Broxmeyer er al. (PNAS, 84:3871-3875, 1987) describe the synergistic myelopoietic actions of purified natural murine CSF-1, recombinant murine IL-3 and recombinant murine GM-CSF. It is shown that individual CSFs, when used at low concentrations, can stimulate the cycling rates and numbers of hematopoietic progenitor cells in the marrows of mice pretreated with human lactoferrin when the CSFs are combined with low dosages of a different CSF.

Rifkin et al. (Behring Inst. Mitt. 83:125-133; August 1988) describe administration of GM-CSF to treat patients with bone marrow failure. The authors report a dose-dependent increase in the total leukocyte, granulocyte and eosinophil counts. They also observed a decrease in mean serum cholesterol level when GM-CSF was administered by continuous infusion but not when it was given as an intravenous bolus. Nimer et al. (JAMA 260, No. 22, 3297-3300, Dec. 1988) also observed a serum cholesterol lowering activity of GM-CSF.

This invention comprises use of a therapeutically effective amount of macrophage colony stimulating factor (M-CSF) and/or granulocyte macrophage colony stimulating factor (GM-CSF) and/or interleukin-3 (IL-3) in admixture with a pharmaceutically acceptable carrier for the preparation of a pharmaceutical composition for improving the lipoprotein profile in a mammal, for reducing total cholesterol levels in a mammal and/or for reducing atherosclerotic lesions in a mammal afflicted with such lesions.

The therapeutically effective amounts of M-CSF, GM-CSF and IL-3 are in the ranges of about 1-200 µg/kg/day, about 1-50 µg/kg/day and about 1-50 µg/kg/day, respectively. When M-CSF and IL-3 and/or GM-CSF are used conjointly or serially, the therapeutically effective amount of each is 1-50 µg/kg/day.

The invention provides the use of M-CSF, GM-CSF, and IL-3 for the preparation of a pharmaceutical composition for improving the lipoprotein-cholesterol profile of a patient by increasing the HDL/LDL ratio. As used herein the term "improved lipoprotein cholesterol profile" is one in which the HDL cholesterol/LDL cholesterol ratio is increased. Optimally, the HDL cholesterol level is raised and the LDL cholesterol level is lowered.

More specifically, the invention concerns the preparation of a pharmaceutical composition of a therapeutically effective amount of M-CSF and/or GM-CSF and/or IL-3 solely, or any combination, used conjointly or serially and in admixture with a pharmaceutically acceptable carrier, for improving the lipoprotein-cholesterol profile, whereby HDL levels may be raised and LDL levels may be lowered, or levels of total serum cholesterol may be lowered in mammals.

Because HDLs scavenge serum cholesterol and provide the vehicle by which serum cholesterol is either put to constructive use or modified for safe and efficient removal, then an increased level of HDLC in the patient indicates improved removal of total cholesterol. Improving removal of cholesterol from the bloodstream of the mammal reduces the build-up of atherosclerotic plaques and so reduces the risk of coronary heart disease to that patient. Additionally, the preparations of the present invention reduce the LDLC levels, as well as the levels of the other lipoproteins and the lipids in mammals. Thus, the preparations of this invention may lower levels of total cholesterol and other lipids in mammals.

The invention further provides preparations for removing atherosclerotic lesions even after they have formed. As such, it is contemplated that the preparations of this invention may effect the regression of atherosclerotic lesions, which increase the risk of coronary heart disease. Thus, the invention provides preparations for both the improvement of a mammal's lipoprotein profile, as well as for reversing the formation of atherosclerotic lesions.

Furthermore, it is contemplated that other cholesterol reducing agents, e.g. lovastatin, cholestipol, lopid, cholestyramine, probucol, may be used conjointly with M-CSF and/or IL-3 and/or GM-CSF for the preparation of pharmaceutical compositions and/or as anti-cholesterolic agents to both improve the lipoprotein profile and cause a regression of atherosclerotic lesions in mammals.

The pharmaceutical compositions of the present invention comprise M-CSF (also referred to as CSF-1) and/or GM-CSF and/or IL-3 in combination with a pharmaceutically acceptable excipient. M-CSF produces colonies that contain primarily macrophages. Long form M-CSF (LCSF-1) has been specifically described by Wong et al., Science, 235:1504-1508 (1987). Its production by recombinant DNA techniques is described in WO87/06954. See also EP 0,261,592; WO88/03173; and EP-A 0,276,551.

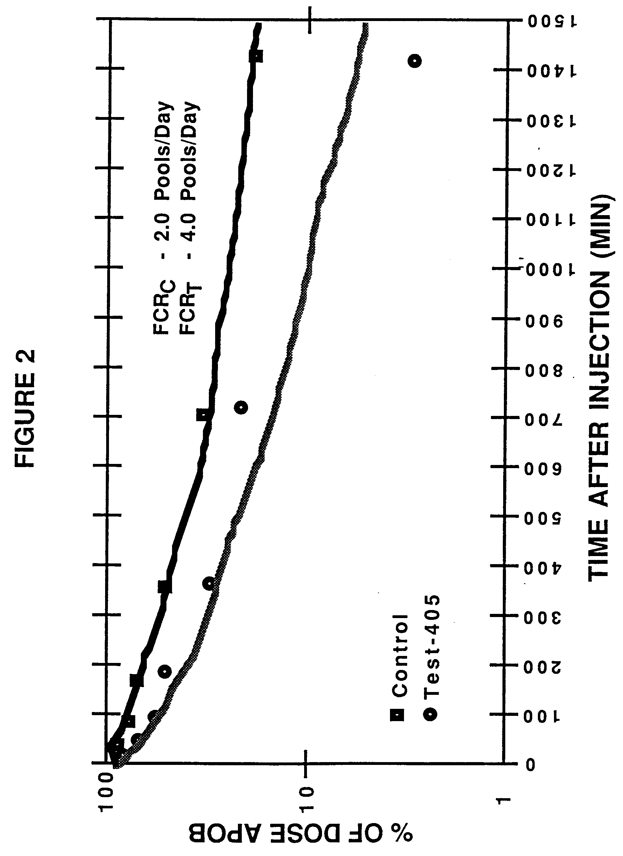

A truncated version of CSF-1 is described in WO86/04607. Kawasaki et al., Science. 230:291-296 (1985) describe the cDNA sequence encoding that truncated protein. Variations on that truncated version having deletions or substitutions of hydrophobic amino acids characteristic of a transmembrane region are described in EP-A 0,249,477. Methods for purifying CSF-1 proteins from natural sources are described in GB 2,016,477; and WO86/04587. (See also, S.K. Das et al., Blood, 58:630 (1981); Stanley et al., J. Biolog. Chem., 252:4305-4312 (1977); Stanley et al., J. Immunol. Methods, 42:253-284 (1981); Stanley, Methods in Enzymology, 116:564-587 (1985); and references cited in WO86/04587)
The M-CSF proteins employed in this invention include the natural proteins, recombinant versions thereof, and derivatives and analogues thereof, which may contain amino acid deletions, substitutions and/or insertions, but which retain the characteristic biological activity of M-CSF; which is the ability to proliferate the growth of cells predominantly of the monocyte/macrophage lineage in the standard bone marrow assay of Wong, et al., Science, 235:1504-1508 (1987), and are encoded by DNAs capable of hybridizing, especially under stringent conditions, to DNAs for the naturally occurring version, as shown in Figure 2 of Wong, et al. Also included, of course, are naturally-occurring isotypes or allelic variations in the protein or its coding sequence as occur in different members of a species.

Another pharmaceutical composition or use comprises primate, or more preferably human IL-3, which may be administered solely or conjointly with GM-CSF. IL-3 stimulates proliferation of a broad spectrum of cell line precursors of the immune system. See Lopez, et al., Proc.Nat.Acad.Sci., USA, 84:2761-2765 (1987).

IL-3 has been specifically described by Yang, et al., Cell, 47:3-10 (1986). Its production by recombinant DNA techniques and methods for purifying IL-3 from the human T-cell line and recombinant primate IL-3 are described therein and in WO88/00598. See also Emerson, et al., J.Clin.Invest., 76:1286-1290 (1985). GM-CSF has been specifically described, its production by recombinant DNA techniques has been disclosed and its purification of the natural GM-CSF from the human T-cell line and the recombinant protein from primate cells has been set forth. Wong, et al., Cancer Cells 3/Growth Factors and Transformation, pps 235-242 (1985), WO86/00639. IL-3's interaction with GM-CSF is described in Donahue, et al., Science, 241:1820-1823 (1988) and Leary, et al., Blood, 70:1343-1348 (1987).

The IL-3 and GM-CSF proteins employed in this invention include the natural primate and human proteins, recombinant versions thereof, and derivatives and analogues thereof, which may contain amino acid deletions, substitutions and/or insertions, but which retain the characteristic biological activity and are encoded by DNAs capable of hybridizing, especially under stringent conditions, to DNAs for the naturally occurring isotypes or allelic variations in the protein or its coding sequence as occur in different members of a species, or which would so hybridize but for the use of synonymous codons (regardless of host cell).

The pharmaceutical compositions may be administered parenterally, e.g. intravenously, or subcutaneously. When parenterally administered, the therapeutic preparations for use in this invention may be in the form of non-pyrogenic, sterile, parenterally acceptable aqueous solutions.

A therapeutically effective dose of M-CSF, i.e. an amount sufficient to increase the HDL cholesterol/LDL cholesterol ratio, is in the range of 1-200 micrograms µg/kg/day. The presently preferred dose is in the range of about 5-150 µg/kg/day. A therapeutically effective dose of IL-3, i.e. an amount sufficient to reduce total serum cholesterol, is in the range of about 1-50 µg/kg/day. The presently preferred dose is in the range of about 1-30 µg/kg/day. Likewise, a therapeutically effective dose of GM-CSF is in the range of about 1-50 µg/kg/day and the presently preferred dose is in the range of about 1-30 µg/kg/day. Reduced amounts of individual components may be used when combinations of those components are used.

The present invention may involve a series of administrations of the pharmaceutical compositions. Such a series may take place over a period of about 7-14 days and involve various combinations of M-CSF, IL-3 and/or GM-CSF, administered conjointly or serially. Additionally, it is contemplated that the compositions, agents and uses of the present invention may be used in chronic treatment for maintaining an acceptable lipoprotein cholesterol profile in a mammal.

The actual dosage regimen utilized in the compositions and uses of the present invention will be determined by the attending physician considering various factors which modify the action of drugs, e.g., the condition, body weight, sex, and diet of the patient, the severity of the condition, time and method of administration and other clinical factors.

The following Examples are illustrative of the improvements that may be observed in the lipoprotein profiles of mammals in response to M-CSF administration alone.

### Example 1

The patient was a normal cynomolgus macaque monkey, weighing approximately 5 kg, and having a cholesterol level as indicated in Table I below. M-CSF was administered by intravenous bolus injection at a dose of 100 µg/kg/day on days 1-15, 45-59, and 73-87.

This animal demonstrated a persistent reduction in serum cholesterol and LDL cholesterol, and an actual rise in HDL cholesterol.

**TABLE I**

| Day | Cholesterol | HDL Cholesterol | LDL Cholesterol |
|---|---|---|---|
| 1 | 145 | 66 | 70 |
| 27 | 129 | 68 | 54 |
| 28 | 127 | 79 | 41 |
| 73 | 131 | 71 | 54 |

### Example 2

The patient was a normal cynomolgus macaque monkey weighing approximately 5 kg, and having a cholesterol level as indicated in Table II below. M-CSF was administered by intravenous bolus injection at a dose of 50 µg/kg/day on days 1-15, 29-34, and 59-73.

This animal also demonstrated a reduction in serum cholesterol and LDLC and a rise in HDLC.

**TABLE II**

| Day | Cholesterol | HDL Cholesterol | LDL Cholesterol |
|---|---|---|---|
| 1 | 142 | 79 | 56 |
| 23 | 142 | 80 | 56 |
| 27 | 140 | 85 | 50 |
| 29 | | 87 | |
| 85 | 123 | 80 | 36 |

### Example 3

A female New Zealand white rabbit was cannulated with an INFUSAID® silicon rubber catheter on one facial vein and a MICRO-RENETHANE® catheter on the opposite jugular vein. The catheters were subcutaneously routed and exited between the scapula. A jacket that held an ABBOTT PARKER® pump was placed on the rabbit and the INFUSAID® catheter was connected to the pump. The other catheter was heperin-locked for blood sampling.

After a one week recovery period, the rabbits were started on a constant infusion of M-CSF at 100 mg/Kg/day. Blood samples for cholesterol analysis were drawn on days 0, 8 and 15.

This animal demonstrated a consistent reduction in total cholesterol levels, LDL cholersterol levels, and a rise in HDL cholesterol levels.

**TABLE III**

| Day | Cholesterol | HDL Cholesterol | LDL Cholesterol |
|---|---|---|---|
| 0 | 134 | 21 | 76 |
| 8 | 108 | 26 | 71 |
| 15 | 92 | 27 | 50 |

### Example 4

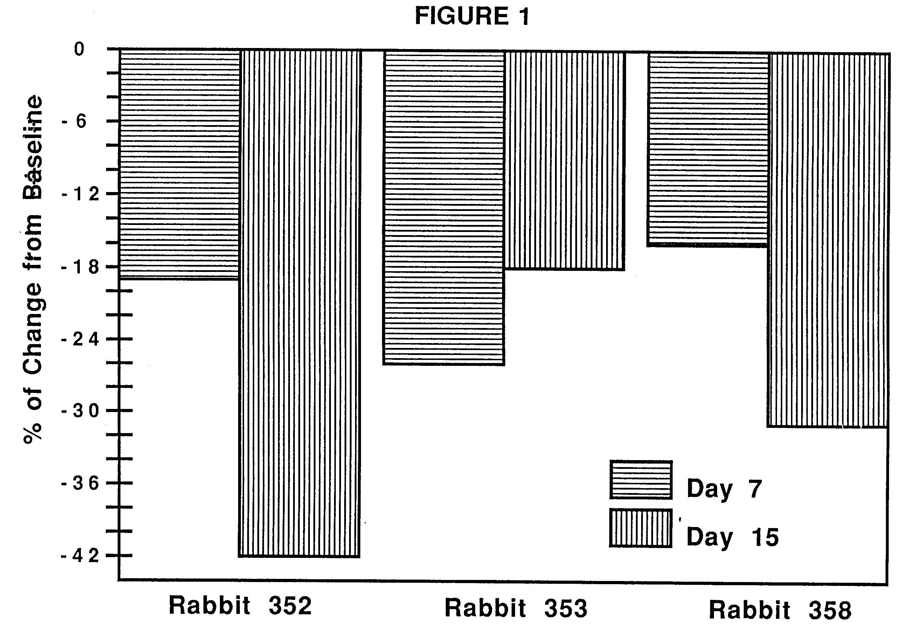

M-CSF was administered to three New Zealand white rabbits either by a constant intravenous infusion after the animals had been prepared as set forth in Example 3 above, or by subcutaneous injection, for a period of 7 days. Plasma cholesterol levels of all three decreased, up to 42% from baseline values following treatment. A decrease in the levels of low density lipoprotein cholesterol accounted for the majority of the observed decrease in total serum cholesterol values. High density lipoprotein cholesterol levels were decreased slightly or in some cases even increased over baseline values. A trend toward a continuing decline in cholesterol levels was seen following termination of dosing. See Fig. 1.

### Example 5

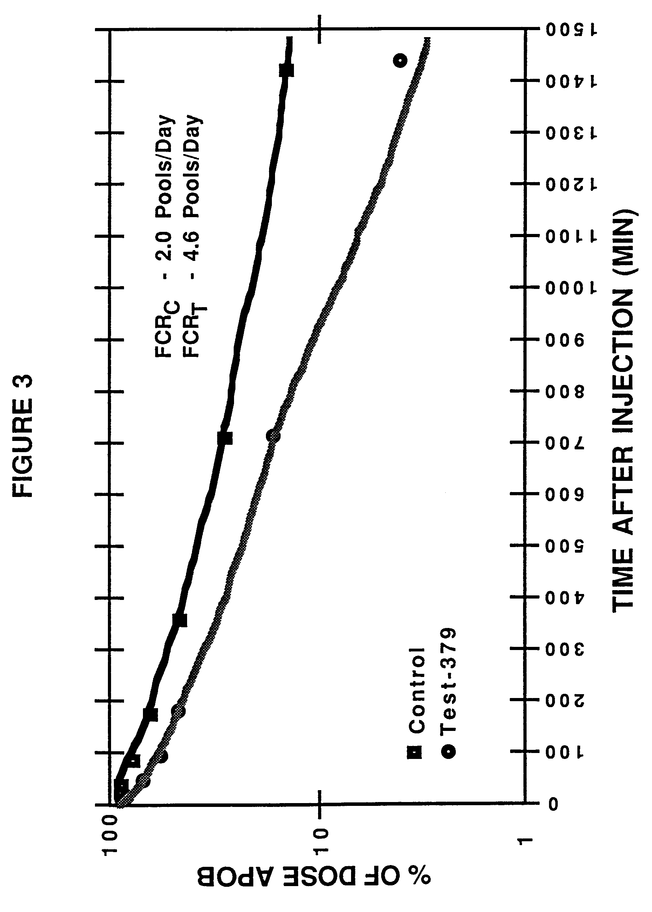

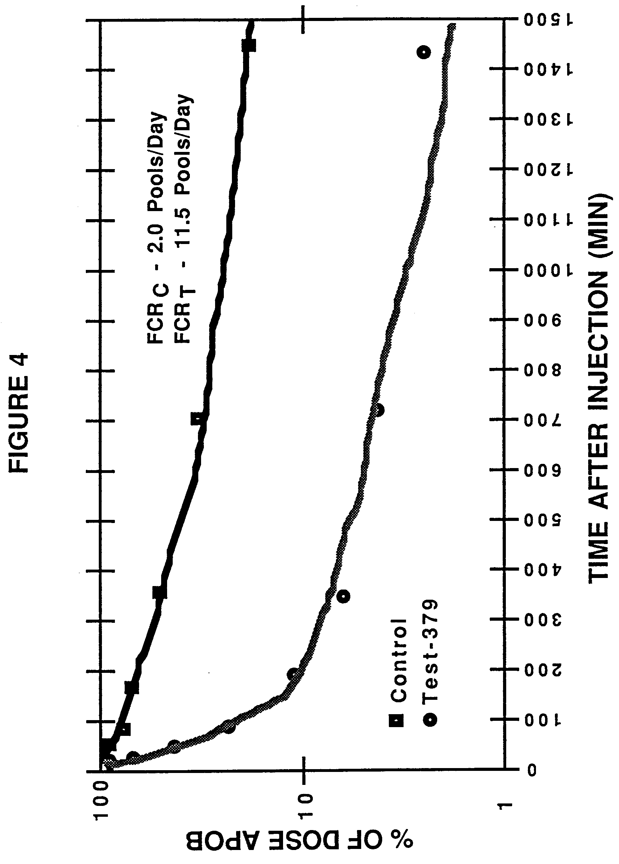

Estimates of the clearance of low density lipoprotein were made in New Zealand white rabbits, using radioiodinated LDL. In these studies radiolabelled LDL was injected into untreated rabbits and into rabbits receiving the dosage regimen set forth in Example 4 above. Blood samples were taken at intervals following injection, and aliquots of plasma were counted for ApoproteinB (ApoB) radioactivity. Plasma ApoB-radioactivity time curves were constructed from these data and are set forth herein in Figures 2, 3, and 4. Referring to those figures, the curves shown therein were fit to a bi-exponential function. The fractional catabolic rate (FRC), pools per day, was derived from the fits of the tracer data. The results indicate that the FCR of rabbits receiving M-CSF is increased, as compared to the FCR of LDL in the control animals.

The following examples are illustrative of the improvements that may be observed in the lipoprotein profiles of mammals in response to IL-3 administration, and in response to IL-3 and GM-CSF, administered conjointly.

### Example 6

The patient was a normal cynomolgus macaque monkey weighing approximately 5 kg, and having a serum cholesterol indicated in Table IV below. IL-3 was administered by intravenous bolus injection at a dose of 20 µg/kg/day on days 1-7 and GM-CSF was then administered by intravenous bolus injection at a dose of 20 µg/kg/day thereafter, on days 8-12.

This animal demonstrated a steady reduction in serum cholesterol between days 1-7 and days 8-18.

**TABLE IV**

| Day | Cholesterol |
|---|---|
| 1 | 118 |
| 7 | 114 |
| 12 | 102 |
| 18 | 101 |

### Example 7

The patient was a normal cynomolgus macaque monkey weighing approximately 5 kg and having a serum cholesterol indicated in Table V. GM-CSF was administered by intravenous bolus injection at a dose of 2 µg/kg/day on days 1-7 and IL-3 was then administered by intravenous bolus injection at a dose of 20 µg/kg/day on days 8-14.

This animal also demonstrates a consistent reduction in serum cholesterol.

**TABLE V**

| Day | Cholesterol |
|---|---|
| 0 | 154 |
| 7 | 138 |
| 14 | 67 |

### Example 8

The patient was a normal cynomolgus macaque monkey weighing approximately 5 kg, and having a serum cholesterol indicated in Table VI. IL-3 was administered by intravenous bolus injection at a dose of 20 µg/kg/day on days 1-7 and GM-CSF was then administered by IV bolus injection at a dose of 2 µg/kg/day on days 8-14.

This animal demonstrates a consistent reduction in total cholesterol levels.

**TABLE VI**

| Day | Cholesterol |
|---|---|
| 0 | 111 |
| 7 | 100 |
| 14 | 93 |

### Example 9

The patient was a normal cynomolgus macaque monkey weighing approximately 5 kg, and having a serum cholesterol indicated in Table VII. GM-CSF was administered by intravenous bolus injection at a dose of 2 µg/kg/day on days 1-7 and IL-3 was then administered by intravenous bolus injection at a dose 20 µg/kg/day on days 8-14.

This animal demonstrates a steady reduction in serum cholesterol during days 1-7 and a significant reduction through days 8-14.

**TABLE VII**

| Day | Cholesterol |
|---|---|
| 1 | 145 |
| 7 | 138 |
| 14 | 113 |

## Claims

1. Use of M-CSF and/or GM-CSF and/or IL-3 in admixture with a pharmaceutically acceptable carrier for the preparation of a pharmaceutical composition for improving the lipoprotein profile in a mammal, for reducing total cholesterol levels in a mammal or for reducing atherosclerotic lesions in a mammal afflicted with such lesions.

## Patentansprüche

1. Verwendung von M-CSF und/oder GM-CSF und/oder IL-3 in Mischung mit einem pharmazeutisch annehmbaren Trägerstoff zur Herstellung einer pharmazeutischen Zusammensetzung zur Verbesserung des Lipoprotein-Profils bei Säugern (einschließlich Mensch), zur Senkung des Gesamtcholesterinspiegels bei Säugern oder zur Verminderung atherosklerotischer Läsionen bei Säugern mit solchen Läsionen.

## Revendications

1. Utilisation du M-CSF (facteur de stimulation de la lignée des macrophages) et/ou du GM-CSF (facteur de stimulation de la lignée des granulocytes-macrophages)et/ou d'IL-3 (interleukine 3) en mélange avec un véhicule pharmaceutiquement acceptable, pour la préparation d'une composition pharmaceutique pour l'amélioration du profil lipoprotéique chez un mammifère, pour la diminution de taux de cholestérol total chez un mammifère ou pour la réduction de lésions athérosclérotiques chez un mammifère souffrant de telles lésions.
